(19) Europäisches Patentamt
European Patent Office
Office européen des brevets

(11) **EP 4 398 263 A1**

(12) **EUROPEAN PATENT APPLICATION**
published in accordance with Art. 153(4) EPC

(43) Date of publication:
**10.07.2024 Bulletin 2024/28**

(21) Application number: **22864775.6**

(22) Date of filing: **11.01.2022**

(51) International Patent Classification (IPC):
*G16H 50/50* (2018.01)   *G16H 30/40* (2018.01)
*G16H 30/20* (2018.01)   *G16H 50/20* (2018.01)
*G06N 20/00* (2019.01)   *A61B 1/04* (2006.01)
*A61B 1/00* (2006.01)

(52) Cooperative Patent Classification (CPC):
**A61B 1/00; A61B 1/04; G06N 20/00; G16H 30/20;
G16H 30/40; G16H 50/20; G16H 50/50**

(86) International application number:
**PCT/KR2022/000434**

(87) International publication number:
**WO 2023/033266 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2021 KR 20210115451**

(71) Applicant: **Dongguk University
Industry-Academic
Cooperation Foundation
Seoul 04620 (KR)**

(72) Inventor: **LIM, Yun Jeong
Goyang-si Gyeonggi-do 10326 (KR)**

(74) Representative: **J A Kemp LLP
80 Turnmill Street
London EC1M 5QU (GB)**

(54) **METHOD FOR PROVIDING DISEASE SYMPTOM INFORMATION BY USING DOMAIN ADAPTATION BETWEEN HETEROGENEOUS CAPSULE ENDOSCOPES**

(57)     Provided is a method of providing disease information by domain adaptation between different types of capsule endoscopes. In a method for providing disease information according to an embodiment of the present invention, a cycle GAN analyzes a first medical image and transforms the same to be like a second medical image, and a disease recognition model predicts disease by analyzing the transformed first medical image. Accordingly, disease recognition models may be trained by domain adaptation between different types of capsule endoscopes, and high recognition performance may be achieved even when a disease recognition model developed through one type of capsule endoscope is used for another type.

Fig 6.

## Description

### Technical Field

The present invention relates to medical diagnosis technology using artificial intelligence, and more specifically, to a method and system of providing disease information using a domain adaptation technique between different types of capsule endoscopes.

### Background Art

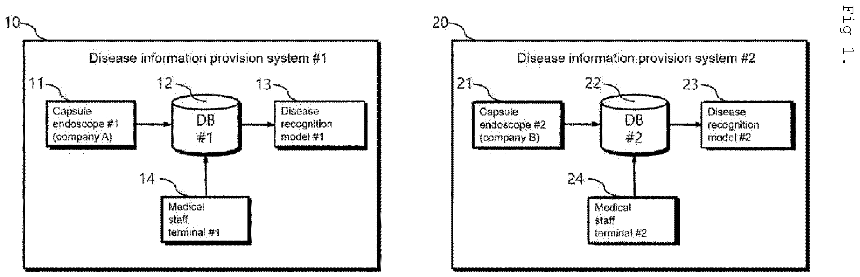

FIG. 1 is a diagram illustrating disease information provision systems using capsule endoscopes. FIG. 1 shows two disease information provision systems (10 and 20), wherein the system (10) on the left and the system (20) on the right use different types of capsule endoscopes.

For example, a capsule endoscope #1 (11) used in the disease information provision system #1 (10) illustrated on the left is a type manufactured/sold by Company A, and a capsule endoscope #2 (21) used in the disease information provision system #2 (20) illustrated on the right is a type manufactured/sold by Company B.

In the disease information provision system #1 (10), capsule endoscopic images obtained by the capsule endoscope #1 (11) are stored in a DB #1 (12). Medical staff use their terminal (14) to annotate the disease information that is their diagnosis results for the capsule endoscopic images stored in the DB #1 (12).

Accordingly, capsule endoscopic images and disease information are stored as data sets in the DB #1 (12). A disease recognition model #1 (13) is an artificial intelligence model that is trained to be capable of predicting disease information by receiving capsule endoscopic images stored in the DB #1 (12).

Accordingly, the disease recognition model #1 (13), when receiving a new capsule endoscopic image, can automatically predict the disease by analyzing the image.

Meanwhile, in the disease information provision system #2 (20), capsule endoscopic images obtained by the capsule endoscope #2 (21) are stored in a DB #2 (22). Then, the medical staff use their terminal (24) to annotate the disease information that is their diagnosis results for the capsule endoscopic images stored in the DB #2 (22).

Accordingly, capsule endoscopic images and disease information are also stored as data sets in the DB #2 (22). A disease recognition model #2 (23) is an artificial intelligence model that is trained to be capable of predicting disease information by receiving capsule endoscopic images stored in the DB #2 (22).

Thereby, the disease recognition model #2 (23), when receiving a new capsule endoscopic image, can also automatically predict the disease by analyzing the image.

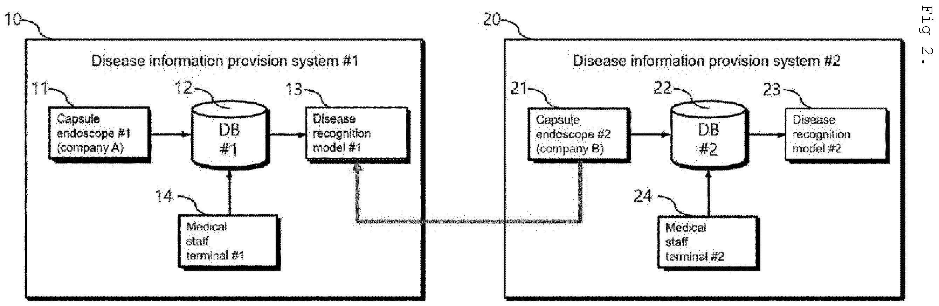

However, since the disease recognition model #1 (13) trains only the capsule endoscopic images obtained by the capsule endoscope #1 (11), when it receives a capsule endoscopic image obtained by the capsule endoscope #2 (21) as illustrated in FIG. 2, the accuracy of disease prediction is very low.

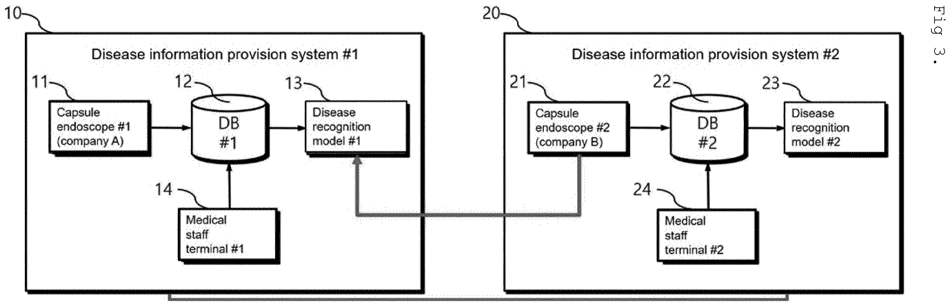

This is also the case of the disease recognition model #2 (23). As illustrated in FIG. 3, even when the disease recognition model #2 (23) receives a capsule endoscope image obtained by the capsule endoscope #1 (11) as illustrated in FIG. 3, the accuracy of disease prediction is very low.

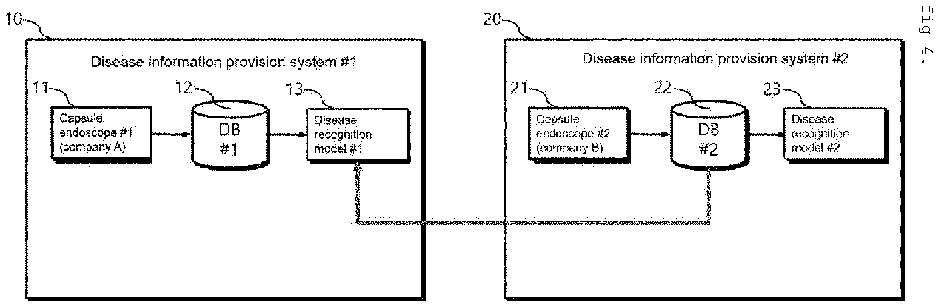

As a way to solve this problem, as illustrated in FIG. 4, retraining the disease recognition model #1 (13) with the data set of capsule endoscopic images and disease information stored in the DB #2 (22) can be contemplated.

However, the capsule endoscopic images obtained by the capsule endoscope #2 (21), which are the capsule endoscopic images stored in the DB #2 (22), are very different in various ways from the capsule endoscopic images obtained by the capsule endoscope #1 (11), which are stored in the DB #1 (12) and used to train the disease recognition model #1 (13). Accordingly, although the performance of the disease recognition model #1 (13) can become higher than the existing one for the DB #2 (22), the performance cannot be expected to be so high, and the performance may be lower than the existing one for the DB #1 (12).

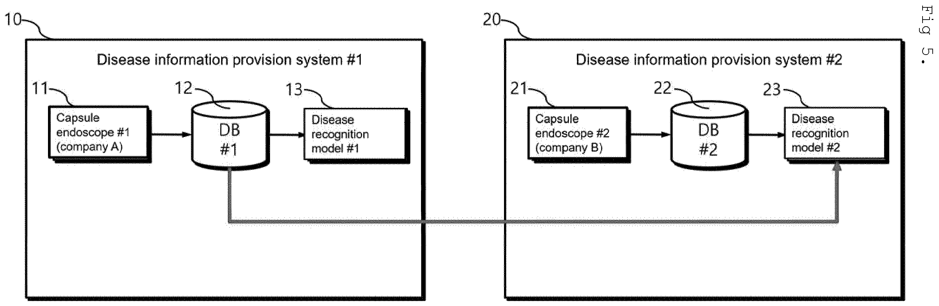

This is also the case of the disease recognition model #2 (23). In other words, as illustrated in FIG. 5, even when the disease recognition model #2 (23) is retrained with the data set of capsule endoscopic images and disease information stored in the DB #1 (12), the training effect is not as good as expected.

[Prior Art Documents]

[Patent Documents]

(Patent Document 1) Korean Patent Application Publication No. 2021-0081805 (July 2, 2021)
(Patent Document 2) Korean Patent Application Publication No. 2020-0131020 (November 23, 2020)
(Patent Document 3) Korean Patent Application Publication No. 2020-0070062 (June 17, 2020)
(Patent Document 4) Korean Patent No. 2115534 (May 20, 2020)

## DISCLOSURE

### Technical Problem

The present invention has been made in order to solve the above-described problems, and an object of the present invention is to provide a method of improving the range and accuracy of disease recognition by training disease recognition models by domain adaptation between different types of capsule endoscopes in a state in which different types of capsule endoscopic DBs have been created.

## Technical Solution

According to one embodiment of the present invention to achieve the above object, a method for providing disease information includes: a step in which a cycle GAN analyzes a first medical image and transforms the same to be like a second medical image; and a step in which a disease recognition model predicts disease by analyzing the transformed first medical image.

The cycle GAN may be trained in the direction of reducing the loss of similarity between a medical image obtained by inversely transforming the transformed first medical image and the first medical image and the loss between the disease predicted by the disease recognition model and ground truth (GT) disease.

The disease recognition model may be firstly trained to predict disease by receiving a second medical image, and secondly trained to predict disease by receiving the first medical image transformed by the cycle GAN.

The second training of the disease recognition model may be performed later than the first training of the disease recognition model. In addition, the training of the cycle GAN and the second training of the disease recognition model may be performed together at the same time.

The first medical image may be a capsule endoscopic image collected using a first type of capsule endoscope, and the second medical image may be a capsule endoscopic image collected using a second type of capsule endoscope.

The first medical image may be a capsule endoscopic image collected using a first type of capsule endoscope at a first medical institution, and the second medical image may be a capsule endoscopic image collected using a first type of capsule endoscope at a second medical institution.

The method for providing disease information according to one embodiment of the present invention may further include a step of pre-processing the first medical image, and the analyzing and transforming of the first medical image may include analyzing the pre-processed first medical image and transforming the same to be like the second medical image.

The step of pre-processing may include matching the shadow area and resolution of the first medical image with those of the second medical image.

Meanwhile, according to another embodiment of the present invention, a system for providing disease information includes: a cycle GAN that analyzes a first medical image and transforms the same to be like a second medical image; and a disease recognition model that predicts disease by analyzing the transformed first medical image.

Meanwhile, according to another embodiment of the present invention, a method for transforming a medical image includes: a step of pre-processing a first medical image; and a step in which a cycle GAN analyzes the pre-processed first medical image and transforms the same to be like a second medical image.

## Advantageous Effects

According to embodiments of the present invention as described above, disease recognition models may be trained by domain adaptation between different types of capsule endoscopes, and high recognition performance may be achieved even when a disease recognition model developed through one type of capsule endoscope is used for another type.

In addition, according to embodiments of the present invention, when not only different types of capsule endoscopes but also the same type of capsule endoscopes are applied in hospitals with different patient disease types, different settings of capsule endoscopes, and different capsule endoscopic data storage methods, it will be possible to ensure high disease recognition performance even for capsule endoscopes secured at other hospitals, and ultimately, it will be possible to create a universal artificial intelligence model whose performance does not significantly deteriorate despite environmental changes such as any type of capsule endoscope, specific patients, or specific hospitals.

Furthermore, according to embodiments of the present invention, medical staff can determine disease by transforming a type of capsule endoscopic image that is unfamiliar to them into a capsule endoscopic image that is familiar to them, indicating that it is possible to improve the convenience of the medical staff and increase the accuracy of diagnosis.

## Brief Description of Drawings

FIG. 1 is a diagram illustrating disease information provision systems using capsule endoscopes.
FIGS. 2 and 3 are diagrams illustrating a situation in which disease is predicted from capsule endoscopic images obtained by different types of capsule endoscopes.
FIGS. 4 and 5 are diagrams illustrating a situation in which a disease recognition model is trained with capsule endoscopic images obtained by different types of capsule endoscopes.
FIG. 6 is a diagram illustrating the structure of a disease information provision system according to one embodiment of the present invention.
FIG. 7 is a diagram illustrating the structure of a disease information provision system according to another embodiment of the present invention.

## Mode for Invention

Hereinafter, the present invention will be described in more detail with reference to the accompanying drawings.

One embodiment of the present invention presents a method of providing disease information by domain adaptation between different types of capsule endoscopes. Specifically, one embodiment of the present invention presents a method of effectively training a disease recognition model with a DB, generated using one type of capsule endoscope, based on a DB generated using another type of capsule endoscope.

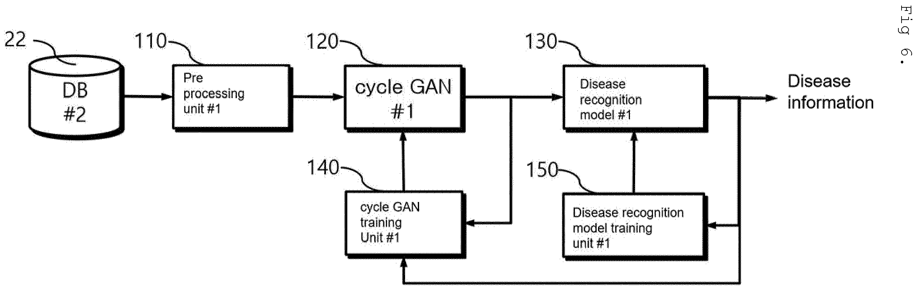

FIG. 6 is a diagram illustrating the structure of a disease information provision system according to one embodiment of the present invention. The disease information provision system according to an embodiment of the present invention corresponds to a system obtained by improving the disease information provision system #1 (10) illustrated in FIG. 1.

As illustrated therein, the disease information provision system according to an embodiment of the present invention includes a pre-processing unit #1 (110), a cycle generative adversarial network (GAN) #1 (120), a disease recognition model #1 (130), a cycle GAN training unit #1 (140), and a disease recognition model #1 training unit #1 (150).

The components of the disease information provision system according to an embodiment of the present invention may be implemented not only in SW, but also in a processor that drives the SW.

A DB #2 (22) is a DB in which the capsule endoscopic images obtained by the capsule endoscope #2 (21) illustrated in FIG. 1 and disease information are stored as a data set.

The pre-processing unit #1 (110) is configured to pre-process the capsule endoscopic image stored in the DB #2 (22), and matches the shadow area and resolution of the capsule endoscopic image of the DB #2 (22) with those of the capsule endoscopic image of a DB #1 (12) (capsule endoscopic image obtained by the capsule endoscope #1 (11)).

In order to transform the capsule endoscopic image of the DB #2 (22) (capsule endoscopic image obtained by the capsule endoscope #2 (21)) into a form like the capsule endoscopic image of the DB #1 (12) (capsule endoscopic image obtained by the capsule endoscope #1 (11)), the pre-processing unit #1 (110) matches the shadow area caused by the difference in the lenses of the capsule endoscopes (11 and 21) and matches the resolution difference between the capsule endoscopes (11 and 21).

The cycle GAN #1 (120) is an artificial intelligence model trained to receive and analyze the capsule endoscopic image of the DB #2 (22) pre-processed by the pre-processing unit #1 (110) and transform the same to be like the capsule endoscopic image of the DB #2 (22) (capsule endoscopic image obtained by the capsule endoscope #1 (11)).

The "capsule endoscopic image obtained by the capsule endoscope #1 (11)" and the "capsule endoscopic image obtained by the capsule endoscope #2 (21)" are different in terms of the degree of blur due to focus difference, brightness due to lighting, image content due to capsule buoyancy and posture control, and the like. The cycle GAN #1 (120) applies a deep learning-based domain adaptation technique to transform the "capsule endoscopic image obtained by the capsule endoscope #1 (11)" to be like the "capsule endoscopic image obtained by the capsule endoscope #2 (21)".

The disease recognition model #1 (130) is an artificial intelligence model that predicts disease by receiving and analyzing the capsule endoscope image of the DB #2 (22), transformed to be like the capsule endoscopic image (obtained by the capsule endoscope #1 (11)) by the cycle GAN #1 (120).

The cycle GAN training unit #1 (140) trains the cycle GAN #1 (120) so that the cycle GAN #1 (120) transforms the capsule endoscopic image of the DB #2 (22) to be like the capsule endoscopic image obtained by the capsule endoscope #1 (11).

To this end, the cycle GAN training unit #1 (140) trains parameters of the cycle GAN #1 (120) in the direction of reducing the loss between the "capsule endoscopic image obtained by inversely transforming the capsule endoscopic image of the DB #2 (22), transformed by the cycle GAN #1 (120)" and the "original capsule endoscopic image of the DB #2 (22), received in the cycle GAN #1 (120)".

In addition, the cycle GAN training unit #1 (140) further uses the prediction result of the disease recognition model #1 (130) to train the cycle GAN #1 (120). Specifically, the cycle GAN training unit #1 (140) trains the cycle GAN #1 (120) in the direction of reducing the loss between the disease predicted by the disease recognition model #1 (130) and the ground truth (GT) disease.

In addition to allowing the cycle GAN #1 (120) to transform the capsule endoscopic image of DB #2 (22) to be like the capsule endoscopic image obtained by the capsule endoscope #1 (11), the cycle GAN training unit #1 (140) trains the cycle GAN #1 (120) to perform capsule endoscopic image transformation so that the disease recognition model #1 (130) is capable of accurately predicting disease.

A total loss function that is used by the cycle GAN training unit #1 (140) may be expressed as follows:

$$L_T = aL_C + (1-a)L_P$$

wherein $L_T$ is the total loss function, $L_C$ is the loss between a capsule endoscopic image obtained by inversely transforming the transformed capsule endoscopic image of the DB #2 (22) and the original capsule endoscopic image of the DB #2 (22), $L_P$ is the loss between the disease predicted by the cycle GAN #1 (120) and GT disease, and a is a weighted value to determine the importance of $L_C$ and $L_P$ in LT.

Meanwhile, the disease recognition model #1 (130) is one trained with the capsule endoscopic images of the DB #1 (12), like the disease recognition model #1 (11) illustrated in FIG. 1.

For the disease recognition model #1 (130) in this state, the disease recognition model training unit #1 (150) trains the disease recognition model #1 (130) using the capsule endoscopic image of the DB #2 (22), transformed to be like the capsule endoscopic image of the DB #1 (12) (obtained by the capsule endoscope #1 (11)) by the cycle GAN #1 (120).

Specifically, the disease recognition model training unit #1 (150) trains the disease recognition model #1 (130) in the direction of reducing the loss between the disease predicted by receiving the capsule endoscopic image of the DB #2 (22), transformed to be like the capsule endoscopic image (obtained by the capsule endoscope #1 (11)) by the cycle GAN #1 (120), and GT disease.

In the training step, the training of the cycle GAN #1 (120) by the cycle GAN training unit #1 (140) and the training of the disease recognition model #1 (130) by the disease recognition model training unit #1 (150) are performed together at the same time.

As a result of training, the cycle GAN #1 (120) is able to transform the capsule endoscopic image of the DB #2 (22) to be close to the capsule endoscopic image obtained by the capsule endoscope #1 (11), and the disease recognition model #1 (130) is able to accurately predict disease information from the capsule endoscopic image transformed by the cycle GAN #1 (120).

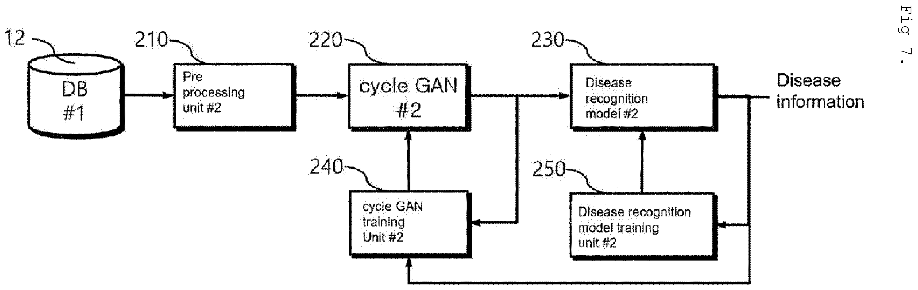

FIG. 7 is a diagram illustrating the structure of a disease information provision system according to another embodiment of the present invention. The disease information provision system according to an embodiment of the present invention corresponds to a system obtained by improving the disease information provision system #2 (20) illustrated in FIG. 1.

As illustrated therein, the disease information provision system according to an embodiment of the present invention includes a pre-processing unit #2 (210), a cycle GAN #2 (220), a disease recognition model #2 (230), a cycle GAN training unit #2 (240), and a disease recognition model training unit #2 (250).

A DB #1 (12) is a DB in which the capsule endoscopic images obtained by the capsule endoscope #1 (11) illustrated in FIG. 1 and disease information are stored as a data set.

The pre-processing unit #2 (210) matches the shadow area and resolution of the capsule endoscopic image stored in the DB #1 (12) with those of the capsule endoscopic image of a DB #2 (22) (capsule endoscopic image obtained by the capsule endoscope #2 (21)).

The cycle GAN #2 (220) is an artificial intelligence model trained to receive and analyze the capsule endoscopic image of the DB #1 (12) pre-processed by the pre-processing unit #2 (210) and transform the same to be like the capsule endoscopic image of the DB #2 (22) (capsule endoscopic image obtained by the capsule endoscope #2 (21)).

The disease recognition model #2 (230) is an artificial intelligence model that predicts disease by receiving and analyzing the capsule endoscope image of the DB #1 (12), transformed to be like the capsule endoscopic image (obtained by the capsule endoscope #2 (21)) by the cycle GAN #2 (220).

The cycle GAN training unit #2 (240) trains the cycle GAN #2 (220) so that the cycle GAN #2 (220) transforms the capsule endoscopic image of the DB #1 (12) to be like the capsule endoscopic image obtained by the capsule endoscope #2 (21) while the accuracy of disease recognition of the disease recognition model #2 (230) is increased.

The disease recognition model #2 (230) is one trained with the capsule endoscopic images of the DB #2 (22), like the disease recognition model #2 (21) illustrated in FIG. 1.

For the disease recognition model #2 (230) in this state, the disease recognition model training unit #2 (250) trains the disease recognition model #2 (230) using the capsule endoscopic image of the DB #1 (12), transformed to be like the capsule endoscopic image of the DB #2 (22) (obtained by the capsule endoscope #2 (21)) by the cycle GAN #2 (220).

As a result of training, the cycle GAN #2 (220) is able to transform the capsule endoscopic image of the DB #1 (12) to be close to the capsule endoscopic image obtained by the capsule endoscope #2 (21), and the disease recognition model #2 (230) is able to accurately predict disease information from the capsule endoscopic image transformed by the cycle GAN #2 (220).

Meanwhile, detailed description of the components of the disease information provision system according to an embodiment of the present invention may be inferred from detailed description of the components of the system illustrated in FIG. 6, and thus is omitted.

So far, the method of providing disease information by domain adaptation between different types of capsule endoscopes has been described in detail by way of preferred embodiments.

The above embodiments present a method of expanding the range of disease recognition by training a disease recognition model, trained with a DB generated using one type of capsule endoscope, based on a DB generated using another type of capsule endoscope.

Meanwhile, the technical idea of the present invention is also applicable to the same type of capsule endoscopes rather than different types of capsule endoscopes. For example, for a disease recognition model A trained with a DB generated using type a of capsule endoscope at medical institution A, the technical idea of the present invention is also applicable to a system that transforms a DB, generated using the same type a of capsule endoscope at medial institution B, by the cycle GAN, and predicts disease by the disease recognition model A.

Patient's disease types, capsule endoscope settings, and capsule endoscopic data storage methods are different between medical institutions. Thus, when the method according to an embodiment of the present invention is applied, high disease recognition performance may be secured even for capsule endoscopic images obtained in other hospitals.

Ultimately, according to an embodiment of the present invention, it is possible to create a universal disease recognition model whose performance does not significantly deteriorate despite environmental changes such as any type of capsule endoscope, specific patients, or specific hospitals.

Meanwhile, it is possible to add a function of providing capsule endoscopic images, transformed by the cycle GAN (120 or 220), as medical information through a medical staff terminal. Accordingly, medical staff will be able to determine disease using capsule endoscopic images with which they are familiar, indicating that it is possible to improve the convenience of the medical staff and increase the accuracy of diagnosis.

Furthermore, the capsule endoscopic images mentioned in the above embodiments are merely an example of medical images. It is natural that the technical idea of the present invention is applicable not only to endoscopic images other than capsule endoscopic images, but also to medical images other than endoscopic images.

Meanwhile, it is natural that the technical idea of the present invention is also applicable to a computer-readable recording medium containing a computer program to perform the functions of the system and method according to the embodiment of the present invention. Additionally, the technical ideas according to various embodiments of the present invention may be implemented in the form of computer-readable code recorded on a computer-readable recording medium. The computer-readable recording medium may be any data storage device that can be read by a computer and can store data. For example, the computer-readable recording medium may be a read only memory (ROM), a random access memory (RAM), a CD-ROM, a magnetic tape, a floppy disk, an optical disk, a hard disk drive, or the like. In addition, a computer-readable code or program stored in the computer-readable recording medium may be transmitted via a network connected between computers.

While preferred embodiments of the present invention have been illustrated and described, the present invention is not limited to the above-described specific embodiments. It is natural that various modified implementations can be made by a person skilled in the art without departing from the scope of the present invention as claimed in the appended claims, and such modified implementations should not be understood as being separate from the technical idea or prospect of the present invention.

[Description of Reference Numerals]

110, 210: pre-processing unit
120, 220: cycle generative adversarial network (GAN)
130, 230: disease recognition model
140, 240: cycle GAN training unit
150, 250: disease recognition model training unit

**Claims**

1. A method for providing disease information comprising:

   a step in which a cycle GAN analyzes a first medical image and transforms the same to be like a second medical image; and
   a step in which a disease recognition model predicts disease by analyzing the transformed first medical image,
   wherein the disease recognition model is firstly trained to predict disease by receiving a second medical image, and is secondly trained to predict disease by receiving the first medical image transformed by the cycle GAN, after the first training, and
   training of the cycle GAN and the second training of the disease recognition model are performed at the same time.

2. The method according to claim 1, wherein the cycle GAN is trained in a direction of reducing a loss between a medical image obtained by inversely transforming the transformed first medical image and the first medical image and a loss between the disease predicted by the disease recognition model and ground truth (GT) disease.

3. The method according to claim 2, wherein the first medical image is a capsule endoscopic image collected using a first type of capsule endoscope, and the second medical image is a capsule endoscopic image collected using a second type of capsule endoscope.

4. The method according to claim 2, wherein the first medical image is a capsule endoscopic image collected using a first type of capsule endoscope at a first medical institution, and the second medical image is a capsule endoscopic image collected using a first type of capsule endoscope at a second medical institution.

5. The method according to claim 2, further comprising a step in which a pre-processing unit pre-processes the first medical image, wherein the analyzing and transforming the first medical image comprises analyzing the pre-processed first medical image and transforming the same to be like the second medical image.

6. The method according to claim 5, wherein the pre-processing the first medical image comprises matching a shadow area and resolution of the first medical image with those of the second medical image.

7. A system for providing disease information comprising:

a cycle GAN that analyzes a first medical image and transforms the same to be like a second medical image; and
a disease recognition model that predicts disease by analyzing the transformed first medical image,
wherein the disease recognition model is firstly trained to predict disease by receiving a second medical image, and is secondly trained to predict disease by receiving the first medical image transformed by the cycle GAN, after the first training, and
training of the cycle GAN and the second training of the disease recognition model are performed at the same time.

8. A method for transforming a medical image comprising:

a step in which a pre-processing unit pre-processes a first medical image; and
a step in which a cycle GAN analyzes the pre-processed first medical image and transforms the same to be like a second medical image,
wherein the disease recognition model is firstly trained to predict disease by receiving a second medical image, and is secondly trained to predict disease by receiving the first medical image transformed by the cycle GAN, after the first training, and
training of the cycle GAN and the second training of the disease recognition model are performed at the same time.

Fig 1.

EP 4 398 263 A1

Fig 2.

Fig 3.

fig 4.

Fig 5.

Fig 6.

Fig 7.

## INTERNATIONAL SEARCH REPORT

| International application No. |
| --- |
| **PCT/KR2022/000434** |

**A. CLASSIFICATION OF SUBJECT MATTER**

**G16H 50/50**(2018.01)i; **G16H 30/40**(2018.01)i; **G16H 30/20**(2018.01)i; **G16H 50/20**(2018.01)i; **G06N 20/00**(2019.01)i; **A61B 1/04**(2006.01)i; **A61B 1/00**(2006.01)i

According to International Patent Classification (IPC) or to both national classification and IPC

**B. FIELDS SEARCHED**

Minimum documentation searched (classification system followed by classification symbols)

G16H 50/50(2018.01); A61B 5/00(2006.01); G06K 9/00(2006.01); G06N 3/04(2006.01); G06N 3/08(2006.01); G06V 10/40(2022.01); G16H 30/20(2018.01); G16H 30/40(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 사이클 GAN(cycle GAN), 의료영상(medical image), 기계학습(machine learning), 캡슐내시경(capsule endoscope), 도메인적응(domain adaptation)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2021-0016171 A (DONGGUK UNIVERSITY INDUSTRY-ACADEMIC COOPERATION FOUNDATION et al.) 15 February 2021 (2021-02-15) See paragraphs [0001]-[0062]; and figures 1-9. | 1,10 |
| Y | | 2-9,11-12 |
| Y | KR 10-2021-0030669 A (INHA UNIVERSITY RESEARCH AND BUSINESS FOUNDATION) 18 March 2021 (2021-03-18) See paragraphs [0013]-[0031]; and figure 3. | 2-9,11-12 |
| A | KR 10-2020-0093424 A (STRADVISION, INC.) 05 August 2020 (2020-08-05) See paragraphs [0048]-[0091]; and figures 3-4. | 1-12 |
| A | KR 10-2021-0098381 A (KOREA UNIVERSITY RESEARCH AND BUSINESS FOUNDATION) 10 August 2021 (2021-08-10) See paragraphs [0034]-[0083]; and figures 3-6. | 1-12 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **26 May 2022** | **26 May 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2022/000434** |

### C.    DOCUMENTS CONSIDERED TO BE RELEVANT

| Category* | Citation of document, with indication, where appropriate, of the relevant passages | Relevant to claim No. |
| --- | --- | --- |
| A | KR 10-2039138 B1 (LUNIT INC.) 31 October 2019 (2019-10-31)<br>See paragraphs [0030]-[0077]; and figures 2-8. | 1-12 |

Form PCT/ISA/210 (second sheet) (July 2019)

**INTERNATIONAL SEARCH REPORT**
Information on patent family members

International application No.

**PCT/KR2022/000434**

| Patent document cited in search report | | | Publication date (day/month/year) | Patent family member(s) | | | Publication date (day/month/year) |
|---|---|---|---|---|---|---|---|
| KR | 10-2021-0016171 | A | 15 February 2021 | KR | 10-2261408 | B1 | 09 June 2021 |
| KR | 10-2021-0030669 | A | 18 March 2021 | KR | 10-2261111 | B1 | 04 June 2021 |
| | | | | WO | 2021-049784 | A2 | 18 March 2021 |
| | | | | WO | 2021-049784 | A3 | 06 May 2021 |
| KR | 10-2020-0093424 | A | 05 August 2020 | CN | 111489285 | A | 04 August 2020 |
| | | | | EP | 3686849 | A1 | 29 July 2020 |
| | | | | JP | 2020-119553 | A | 06 August 2020 |
| | | | | JP | 6886201 | B2 | 16 June 2021 |
| | | | | KR | 10-2372702 | B1 | 10 March 2022 |
| | | | | US | 10373023 | B1 | 06 August 2019 |
| KR | 10-2021-0098381 | A | 10 August 2021 | | None | | |
| KR | 10-2039138 | B1 | 31 October 2019 | KR | 10-2039138 | B1 | 31 October 2019 |
| | | | | KR | 10-2095684 | B1 | 31 March 2020 |
| | | | | US | 2020-0321118 | A1 | 08 October 2020 |

Form PCT/ISA/210 (patent family annex) (July 2019)

# EP 4 398 263 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20210081805
- KR 20200131020
- KR 20200070062
- KR 2115534